# EUROPÄISCHE PATENTANMELDUNG

(11) **EP 2 992 911 A1**
(43) Veröffentlichungstag der Anmeldung: **09.03.2016**
(21) Anmeldenummer: 14183201.4
(22) Anmeldetag: 02.09.2014
(51) Int. Cl.: A61M 1/00, A61G 7/05

(54) **Aufhängevorrichtung eines Drainagebehälters**

(71) Anmelder: Medela Holding AG, 6340 Baar (CH)
(72) Erfinder: Giezendanner, Charles, 6443 Morschach (CH); Bächler, Cornel, 6038 Gisikon (CH); Ehlert, Hilmar, 602 Hergiswil (CH)
(74) Vertreter: Clerc, Natalia

(57) **Zusammenfassung**

Eine Aufhängevorrichtung eines Drainagebehälters (1), welcher zum Auffangen von abgesaugter Körperflüssigkeit dient, weist auf einer ersten Seite eine erste Befestigungseinheit (31) zum Anhängen und Befestigen des Drainagebehälters (1) an die Aufhängevorrichtung auf. Auf einer zweiten Seite ist eine zweite Befestigungseinheit (500, 51) zum Anhängen und Befestigen der Aufhängevorrichtung an eine Schiene vorhanden. Die erste Befestigungseinheit (31) ist in einem ersten Befestigungsteil (3) und die zweite Befestigungseinheit (500, 51) in einem zweiten Befestigungsteil (5) angeordnet. Die Aufhängevorrichtung umfasst ferner ein Wägeteil (4) mit einem Wägeelement (6), wobei das Wägeteil (4) mit dem ersten Befestigungsteil (3) und dem zweiten Befestigungsteil (5) verbunden ist oder verbindbar ist, um mit dem Wägeelement (6) eine relative Verschiebung zwischen dem ersten und dem zweiten Befestigungsteil (3, 5) zu messen, so dass eine Füllmenge der abgesaugten Körperflüssigkeit im Drainagebehälter (1) bestimmbar ist. Die erfindungsgemässe Aufhängevorrichtung ermöglicht mit einfachen Mitteln eine Bestimmung einer Füllmenge eines Drainagebehälters, insbesondere eines Drainagebehälters mit Drainagebeutel.

## Beschreibung

### TECHNISCHES GEBIET

Die vorliegende Erfindung betrifft eine Aufhängevorrichtung eines Drainagebehälters, eine Befestigungseinrichtung einer derartigen Aufhängevorrichtung, ein Befestigungsteil einer derartigen Aufhängevorrichtung sowie ein Drainagesystem mit einer derartigen Aufhängevorrichtung.

### STAND DER TECHNIK

Drainagebehälter, auch Fluidsammelbehälter genannt, sind Auffangbehälter für Körperflüssigkeiten, welche beim Absaugen im Rahmen medizinischer und kosmetischer Behandlungen anfallen. Die Körperflüssigkeit wird mittels einer Vakuumpumpe über eine patientenseitige Drainageleitung oder Saugleitung in den Behälter geleitet. Der Behälter weist hierfür einen Anschluss für die patientenseitige Drainageleitung und einen Anschluss für die Saug- oder Vakuumpumpe oder für ein Zentralvakuum des Spitals auf. Der Drainagebehälter kann ein Einmalgebrauch-Behälter sein oder mindestens Teile davon können mehrfach verwendet werden.

WO 2007/085100 und WO 2013/177716 offenbaren Drainagebehälter mit einem Aussenbehälter, einem diesen Aussenbehälter dichtend verschliessenden Deckel und einem Beutel, welcher im Aussenbehälter aufgenommen ist und dicht mit dem Deckel verbunden ist. Deckel und Beutel lassen sich gemeinsam vom Aussenbehälter entfernen. Diese Behälter lassen sich mittels einer Schnappverbindung mit einer Aufhängevorrichtung verbinden, welche wiederum an einer Schiene befestigbar ist. Die Schiene ist vorzugsweise eine Wandschiene des Spitals oder der Pflegestation oder eine Bettschiene. Diese Aufhängevorrichtung weist einen Grundkörper auf, welcher auf einer Seite mit einer federbelasteten Klammer zum Einklemmen der Schiene und auf der gegenüberliegenden Seite mit einer vertikal verlaufenden Nut versehen ist. Der Aussenbehälter weist einen entsprechenden in vertikaler Richtung verlaufenden Nutstein auf, welcher in die Nut einschiebbar ist. Der Deckel mit dem Beutel ist für den einmaligen Gebrauch bestimmt und wird anschliessend entsorgt. Der Drainagebehälter lässt sich mehrfach verwenden, jedoch nur begrenzt. Die Aufhängevorrichtung hingegen wird über mehrere Jahre hinweg gebraucht und auch zur Befestigung von anderen medizinischen Vorrichtungen verwendet. Diese Drainagebehälter sowie diese Aufhängevorrichtungen haben sich in der Praxis bewährt.

Der Füllstand der Drainagebehälter muss regelmässig kontrolliert werden, da er einerseits einen Hinweis auf den Drainageerfolg gibt und andererseits nicht überfüllt werden darf. Diese Kontrolle erfolgt im Stand der Technik visuell durch das Pflegepersonal. Dies ist insbesondere dann erschwert oder verunmöglicht, wenn die Flüssigkeit nicht direkt in einem starren Behälter mit Füllstandsanzeige, sondern in einem Beutel gesammelt wird. Es besteht somit das Bedürfnis, derartige Drainagebehälter mit einer Vorrichtung zur Messung der Füllmenge zu versehen.

In artfremden Gebieten sind Füllmengenbestimmungen bekannt. So beschreibt CN 202599512 U einen Becher mit einem Handgriff, in welchen eine Tasse eingehängt werden kann. Im Handgriff ist ein Wiegesensor vorhanden, um das Gewicht der Tasse und deren Inhalt zu bestimmen.

CN 2018997492 U zeigt einen Krug mit einem fest damit verbundenen Handgriff, welcher wiederum einen Wiegesensor aufweist.

In CN 202158898 U ist eine ringförmige Bodenplatte mit einer Wiegevorrichtung vorhanden, an welcher ein Handgriff angeformt ist. Auf diese Bodenplatte lässt sich ein Krug stellen.

US 2010/0089152 und DE 20 2010 010 088 U offenbaren einen Krug mit Handgriff und einer Bodenplatte mit Wiegevorrichtung.

In US 374 759 und US 2 478 272 werden bewegbare mechanische Anzeigen verwendet, um die Füllmenge eines Behälters anzuzeigen.

### DARSTELLUNG DER ERFINDUNG

Es ist eine Aufgabe der Erfindung, die Bestimmung einer Füllmenge eines Drainagebehälters zu erleichtern.

Diese Aufgabe löst eine Aufhängevorrichtung mit den Merkmalen des Patentanspruchs 1.

Die erfindungsgemässe Aufhängevorrichtung eines Drainagebehälters, welcher zum Auffangen von abgesaugter Körperflüssigkeit dient, weist auf einer ersten Seite eine erste Befestigungseinheit zum Anhängen und Befestigen des Drainagebehälters, vorzugsweise seines Aussenbehälters, an die Aufhängevorrichtung auf und auf einer zweiten Seite eine zweite Befestigungseinheit zum Anhängen und Befestigen der Aufhängevorrichtung an eine Schiene. Die erste Befestigungseinheit ist in einem ersten Befestigungsteil und die zweite Befestigungseinheit in einem zweiten Befestigungsteil angeordnet. Die Aufhängevorrichtung umfasst ein Wägeteil mit einem Wägeelement, wobei das Wägeteil mit dem ersten Befestigungsteil und dem zweiten Befestigungsteil verbunden ist oder verbindbar ist, um mit dem Wägeelement eine relative Verschiebung zwischen dem ersten und dem zweiten Befestigungsteil zu messen, so dass eine Füllmenge der abgesaugten Körperflüssigkeit im Drainagebehälter bestimmbar ist. Die zweite Seite ist vorzugsweise benachbart oder gegenüberliegend zur ersten Seite angeordnet.

Dadurch, dass die Messung in der Aufhängevorrichtung und nicht im Behälter selber stattfindet, lässt sich auch die Füllmenge bzw. das Gewicht des Inhalts eines Beutels genau bestimmen.

Des Weiteren hat dies den Vorteil, dass zeitlich nur begrenzt einsetzbare Behälter nach wie vor kostengünstig hergestellt werden können und die elektronischen Teile in der wiederverwendbaren Aufhängevorrichtung integriert sind. Dies ist insbesondere dann der Fall, wenn die erste Befestigungseinheit zur Erstellung einer bei bestimmungsgemässem Gebrauch der Aufhängevorrichtung lösbaren Verbindung mit dem Drainagebehälter, vorzugsweise mit seinem Aussenbehälter, ausgebildet ist.

Vorzugsweise ist alternativ oder zusätzlich auch die zweite Befestigungseinheit zur Erstellung einer bei bestimmungsgemässem Gebrauch der Aufhängevorrichtung lösbaren Verbindung mit der Schiene ausgebildet.

In einer alternativen Ausführungsform sind jedoch die Aufhängevorrichtung oder mindestens das erste Befestigungsteil und das Wägeteil fest mit dem Drainagebehälter, insbesondere dem Aussenbehälter, verbunden.

In einer bevorzugten Ausführungsform bilden das erste Befestigungsteil und das Wägeteil eine fest miteinander verbundene und bei bestimmungsgemässem Gebrauch der Aufhängevorrichtung nicht zur Trennung voneinander vorgesehene Einheit.

Unter bestimmungsgemässem Gebrauch wird in diesem Text ein Gebrauch verstanden, welcher dazu dient, Körperflüssigkeit eines Patienten abzusaugen und welcher hierzu bedingt, dass der Drainagebehälter korrekt befestigt ist, um diese Aufgabe zu erfüllen. Die Erstellung bzw. die Lösung der Verbindungen kann dabei vor oder nach der Absaugung erfolgen. Eine Trennung während des Absaugens ist vorzugsweise ebenfalls möglich. Zur Erstellung und Trennung der Verbindung werden vorzugsweise keine Werkzeuge benötigt, sondern sie lässt sich vorzugsweise von Hand durchführen.

Vorzugsweise bildet das zweite Befestigungsteil eine bei bestimmungsgemässem Gebrauch der Aufhängevorrichtung lösbare Verbindung mit dem Wägeteil. Dies hat den Vorteil, dass das Befestigungsteil auch zur Befestigung von anderen Vorrichtungen verwendet werden kann. Insbesondere kann ein zweites Befestigungsteil verwendet werden, wie es beispielsweise in WO 2007/085100 beschrieben ist.

In einer anderen Ausführungsform bilden das zweite Befestigungsteil und das Wägeteil eine fest miteinander verbundene und bei Gebrauch nicht zur Trennung voneinander vorgesehene Einheit, wodurch das erste Befestigungsteil, das Wägeteil und das zweite Befestigungsteil eine bei Gebrauch nicht zur Trennung voneinander vorgesehene Einheit bilden. Dies hat die Vorteile, dass die Aufhängevorrichtung trotz des integrierten Wägeelements kompakt ausgebildet und relativ kostengünstig ist und dass auch einzelne Teile nicht verloren gehen können.

Als Wägeelement eignet sich insbesondere eine Wägezelle, ein Federelement mit Wegmessung, ein Piezosensor oder ein Drucksensor mit Linearführung.

In einer bevorzugten Ausführungsform weist das Wägeelement, insbesondere die Wägezelle, einen ersten Bereich auf, welcher fest mit dem ersten Befestigungsteil verbunden ist, wobei das Wägeelement einen zweiten Bereich aufweist, welcher fest mit dem Wägeteil verbunden ist. Dadurch lässt sich der zweite Befestigungsteil relativ beliebig ausbilden, und er lässt sich insbesondere mit einer im Gebrauch lösbaren Verbindung mit dem Wägeteil verbinden.

Vorzugsweise weist das zweite Befestigungsteil einen Überlastschutz auf. Dieser ist durch einen in vertikaler Richtung verschiebbaren federbelasteten Bolzen gebildet, auf welchem das Wägeteil aufliegt. Vorzugsweise oder alternativ weist das zweite Befestigungsteil einen Vorsprung auf, welcher sich bis zum ersten Befestigungsteil erstreckt und welcher bei einer Gewichtsüberlast zur Auflage des ersten Befestigungsteils dient. Diese Ausführungsformen ermöglichen einen Überlastschutz, welcher eine Überlastung des Wägeelements, insbesondere eine Überdehnung von Dehnungsmessstreifen der Wägezelle verhindert.

Um die Gewichtsbestimmung möglichst genau durchführen zu können, weist die Aufhängevorrichtung vorzugsweise eine Zugentlastung auf für einen mit dem Drainagebehälter verbundenen Drainageschlauch, welcher zum Absaugen der Körperflüssigkeit dient. Dieser Drainageschlauch wird vorzugsweise aufgehängt. Vorzugsweise weist die Zugentlastung einen Bügel auf, welcher vom Wägeteil und/oder vom zweiten Befestigungsteil getragen ist und welcher wiederum den Drainageschlauch trägt.

Die Aufhängevorrichtung definiert eine Aufhängeachse zur Aufhängung des Drainagebehälters, wobei in einer bevorzugten Ausführungsform ein Neigungssensor vorhanden ist zur Feststellung eines Winkels zwischen der Aufhängeachse und einer Vertikalen im Raum, in welchem sich die Aufhängevorrichtung befindet. Dadurch lässt sich feststellen, ob die Schiene und somit der Drainagebehälter schief steht, und es lassen sich dadurch entstehende Messfehler elektronisch kompensieren. Alternativ oder zusätzlich kann auch gewarnt werden, wenn der Behälter zu schief hängt und umzukippen droht.

Vorzugsweise weist die Aufhängevorrichtung ein Display zur Anzeige der gemessenen Füllmenge auf, damit das Pflegepersonal, die medizinischen Fachpersonen oder auch die Patienten die Messwerte vor Ort ablesen können. Je nach Ausführungsform ist ein akustischer und/oder optischer Signalgeber vorhanden, welcher vor einer drohenden Überfüllung warnt.

In einer bevorzugten Ausführungsform weist die Aufhängevorrichtung eine Elektronikeinheit zur Übertragung der bestimmten Füllmenge und/oder eines Warnsignals an einen externen Empfänger auf. Dieser externe Empfänger kann die Vakuumpumpe, eine Steuerung des Zentralvakuums oder eine Ventileinheit zum Zentralvakuum sein. Vorzugsweise ist der externe Empfänger ein klinisches Netzwerk. Vorzugsweise werden die gemessenen Werte direkt in die Patientendaten eingetragen und/oder erzeugen ein Warnsignal im Stationsraum des Pflegepersonals.

Es lassen sich auch mehrere Drainagebehälter hintereinander oder nebeneinander schalten, wobei die Messwerte aller Drainagebehälter summiert und auf einem gemeinsamen Display vor Ort angezeigt werden. Die Signale lassen sich alternativ oder zusätzlich auch gemeinsam einem externen Empfänger zuführen.

Die gemessenen Werte lassen sich weiter verarbeiten. So lässt sich der Durchfluss bestimmen. Bei Absaugung an mehreren Stellen des Patienten, z.B. bei Fettabsaugung, lässt sich ein Vergleich zwischen den verschiedenen abgesaugten Flüssigkeitsmengen erstellen.

Teile der Aufhängevorrichtung lassen sich auch einzeln herstellen und vertreiben. Beispielsweise weist eine Befestigungseinrichtung der erfindungsgemässen Aufhängevorrichtung ein erstes Befestigungsteil mit einer ersten Befestigungseinheit zum Anhängen und Befestigen des Drainagebehälters an die Aufhängevorrichtung auf und umfasst ferner ein Wägeteil mit einem Wägeelement. Das Wägeteil ist mit dem ersten Befestigungsteil verbunden, und das Wägeteil ist mit einem zweiten Befestigungsteil zwecks Verbindung mit einer Schiene verbindbar, um mit dem Wägeelement eine relative Verschiebung zwischen dem ersten und zweiten Befestigungsteil zu messen, so dass eine Füllmenge der abgesaugten Körperflüssigkeit im Drainagebehälter bestimmbar ist.

Ferner lässt sich ein zweites Befestigungsteil der erfindungsgemässen Aufhängevorrichtung einzeln herstellen und vertreiben. Dieses zweite Befestigungsteil weist eine zweite Befestigungseinheit auf zur Verbindung der Aufhängevorrichtung an einer Schiene. Es besitzt einen Gewichtsüberlastschutz, wobei der Gewichtsüberlastschutz einen federbelasteten Bolzen zum Tragen des Wägeteils aufweist. Alternativ oder zusätzlich kann ein Vorsprung vorhanden sein, auf welchem das erste Befestigungsteil bei einer Überlastung aufliegt.

In einer bevorzugten Ausführungsform bildet die erfindungsgemässe Aufhängevorrichtung gemeinsam mit einem Drainagebehälter ein Drainagesystem. Der Drainagebehälter weist dabei einen Aussenbehälter, einen Beutel zur Aufnahme im Aussenbehälter und einen den Aussenbehälter dicht verschliessenden Deckel auf. Der Beutel ist dicht mit dem Deckel verbunden und ist gemeinsam mit dem Deckel vom Aussenbehälter entfernbar. Der Aussenbehälter weist ferner eine Befestigungseinheit zur Verbindung mit der ersten Befestigungseinheit des ersten Befestigungsteils auf. Die erfindungsgemässe Aufhängevorrichtung lässt sich jedoch auch mit anderen Arten von Drainagebehältern, insbesondere solchen ohne Beutel, verwenden.

Weitere Ausführungsformen sind in den abhängigen Ansprüchen angegeben.

### KURZE BESCHREIBUNG DER ZEICHNUNGEN

Eine bevorzugte Ausführungsform der Erfindung wird im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung eines erfindungsgemässen Drainagesystems;
- Figur 2: eine Seitenansicht des Drainagesystems gemäss Figur 1;
- Figur 3: eine perspektivische Darstellung eines Deckels mit einem Beutel des Drainagesystems gemäss Figur 1;
- Figur 4: eine Explosionsdarstellung einer Aufhängevorrichtung des Drainagesystems gemäss Figur 1;
- Figur 5: eine perspektivische Darstellung der Aufhängevorrichtung gemäss Figur 4 im zusammen gefügten Zustand;
- Figur 6: eine perspektivische Darstellung der Aufhängevorrichtung gemäss Figur 4 mit einer Zugentlastung und
- Figur 7: eine perspektivische Darstellung der zusammen gefügten Aufhängevorrichtung mit Display.

### BESCHREIBUNG BEVORZUGTER AUSFÜHRUNGSFORMEN

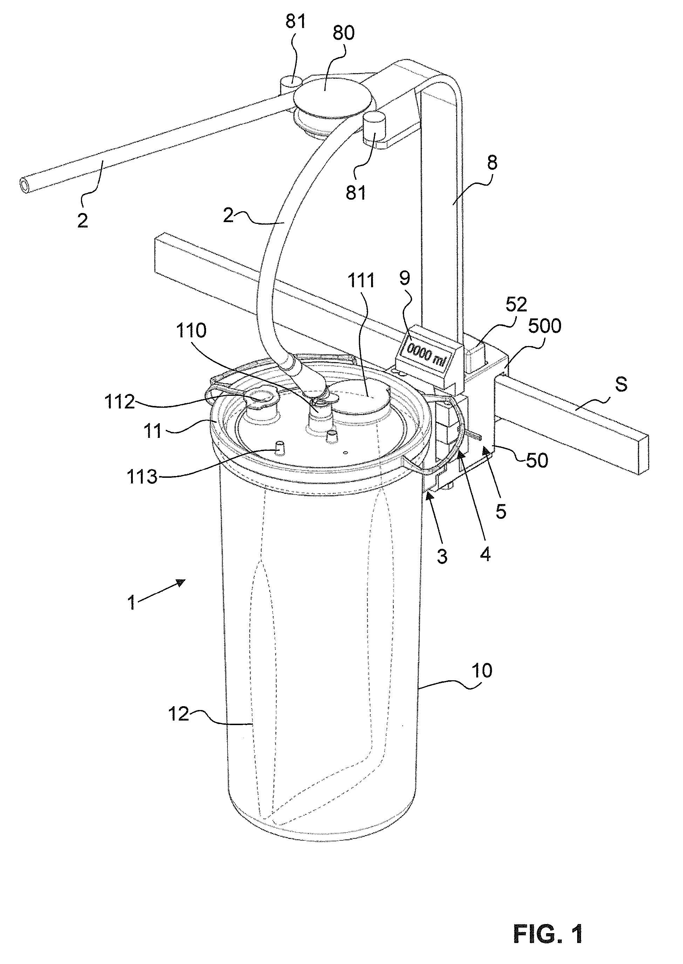

Figur 1 zeigt einen Drainagebehälter 1 mit einem starren, vorzugsweise durchsichtigen Aussenbehälter 10, einem diesen dicht verschliessenden Deckel 11 und einen am Deckel 11 dicht befestigten und im Aussenbehälter 10 aufgenommenen Beutel 12.

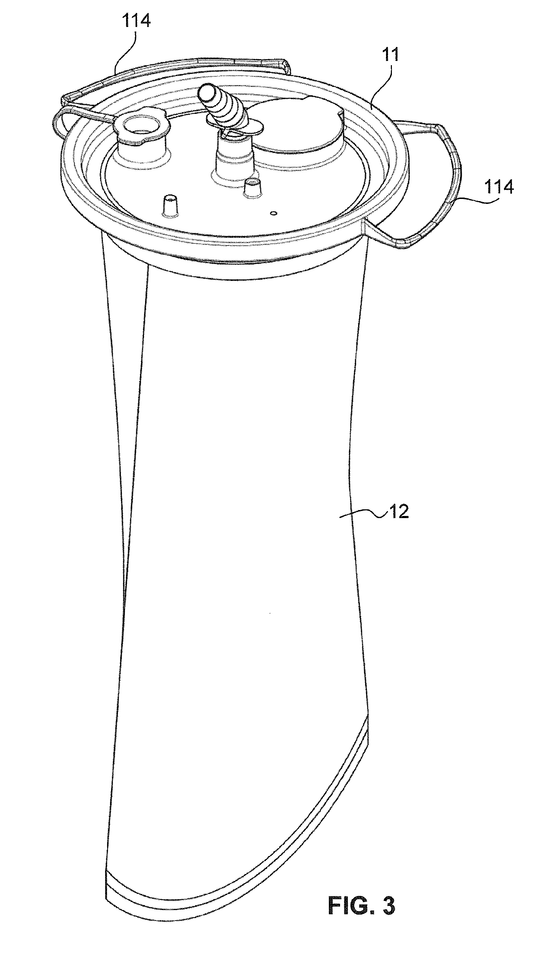

Der Behälter 1 weist die üblichen, für die Verbindung zum Patienten und zur Vakuumquelle notwendigen Mittel auf. Diese sind beispielsweise aus WO 2007/085100 und WO 2013/177716 hinlänglich bekannt. Am Deckel 11 ist ein Drainageanschluss 110 zur Verbindung des Beutelinnenraums mit einem Drainageschlauch 2 vorhanden, wobei der Drainageschlauch 2 zum Patienten führt und die abgesaugte Flüssigkeit in den Behälter leitet. Ferner weist der Deckel 11 vorzugsweise einen seriellen Anschluss 112 auf, damit er in Serie mit einem zweiten Drainagebehälter verbunden werden kann. Die Bezugsziffer 113 bezeichnet einen Befestigungsstutzen, auf welchem üblicherweise Verschlüsse bei Nichtgebrauch aufgesteckt werden. Die Bezugsziffer 111 bezeichnet eine Filterabdeckung, welche ebenfalls zum Beutelinnenraum führt, jedoch einen weiteren Kanal aufweist, der im Zwischenraum zwischen Aussenbehälter und Beutel endet und welcher eine Verbindung zu einem seitlichen Sauganschluss im Aussenbehälter 10 schafft. Der Sauganschluss dient zur Verbindung mit der Vakuumquelle. Er ist hier nicht sichtbar, jedoch aus WO 2013/177716 bekannt. Handgriffe 114 erleichtern das Entfernen des Deckels 11 und des Beutels 12 vom Aussenbehälter 10. Der Deckel 11 mit dem daran befestigten, vorzugsweise angeklebten oder mit ihm verschweissten, Beutel 12 ist in Figur 3 dargestellt.

Der Drainagebehälter 1, genauer der Aussenbehälter 10, ist seitlich an einer Aufhängevorrichtung 3, 4, 5 aufgehängt und befestigt. Die Aufhängevorrichtung 3, 4, 5 lässt sich an eine Schiene S, z.B. an eine Wandschiene oder an eine Bettschiene, hängen und daran befestigen. Die Aufhängevorrichtung 3, 4, 5 trägt ferner einen winkelförmigen Bügel 8, dessen vertikaler Schenkel die restliche Aufhängevorrichtung 3, 4, 5, nach oben überragt und dessen horizontaler Schenkel zum Drainagebehälter 1 zugewandt ist. Vertikal in diesem Text bezieht sich auf die bestimmungsgemässe Gebrauchslage der Vorrichtung.

Am freien Ende des horizontalen Schenkels ist eine Umlenkrolle 80 und zwei diese flankierende Führungsstifte 81 befestigt. Zwischen Umlenkrolle 80 und den Führungsstiften 81 wird der Drainageschlauch gehalten, ohne zusammengepresst zu werden. Der Bügel 8 bildet somit eine Zugentlastung und trägt das Gewicht des von der Umlenkrolle 80 zum Patienten führenden Drainageschlauchs 2.

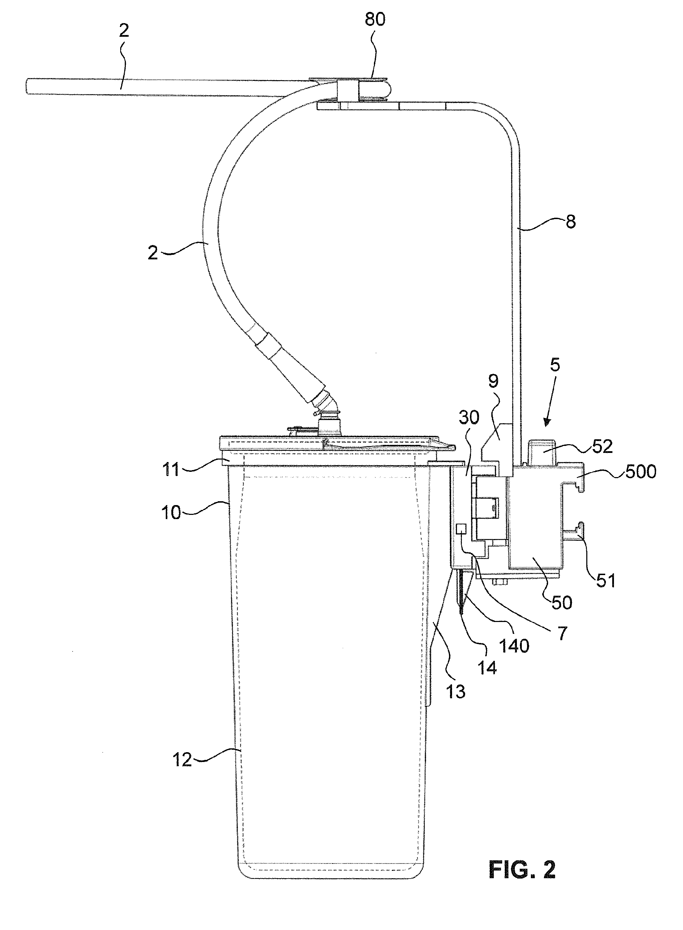

Wie in Figur 2 gut erkennbar ist, weist der Aussenbehälter 10 einen seitlich vorstehenden Befestigungsvorsatz 13 mit einem Nutstein auf. Am Nutstein ist ein Federelement 14 mit einer Rückhaltenase 140 angeordnet, wobei das Federelement eine nach unten gerichtete Verlängerung des vertikal verlaufenden Nutsteins bildet. Auch dies entspricht dem Behälter gemäss WO 2007/085100.

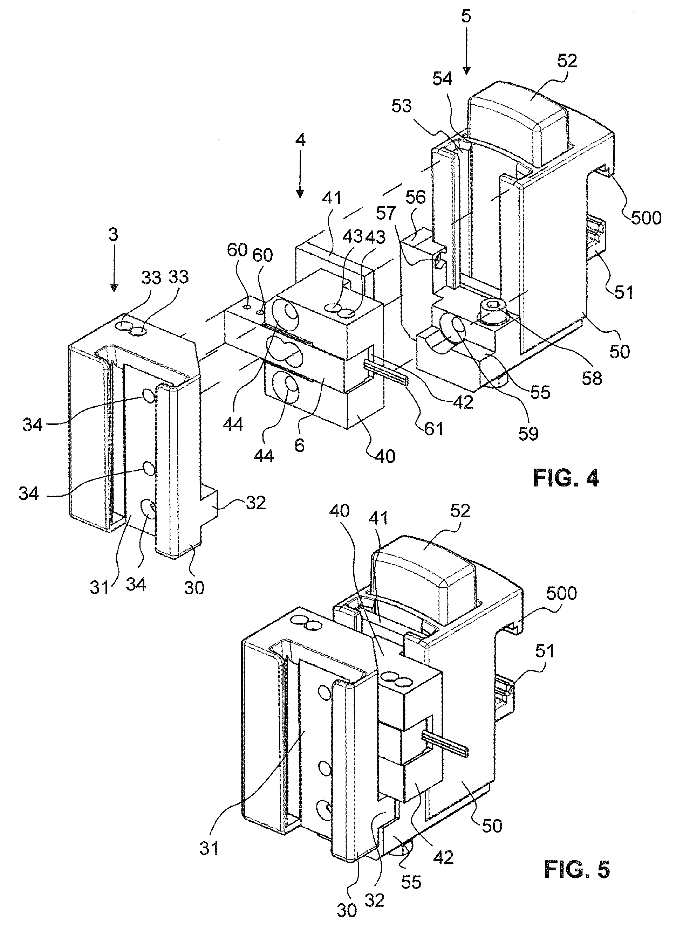

Dieser Nutstein ist in einer ersten Nut 31 (Figur 4) eines behälterseitigen Befestigungsteils 3 der erfindungsgemässen Aufhängevorrichtung eingeschoben und mit der federbelasteten Rückhaltenase 140 in seiner Position fixiert. Dadurch hängt der Behälter 1 an diesem ersten behälterseitigen Befestigungsteil 3. Die erste Nut 31 bildet in diesem Beispiel eine erste Befestigungseinheit.

Die Aufhängevorrichtung weist ferner ein zweites, schienenseitiges Befestigungsteil 5 auf. Dieses weist einen im Wesentlichen quaderförmigen Grundkörper 50 auf, an welchem eine obere feste Klaue 500 angeformt ist. Die Klaue 500 befindet sich auf der dem ersten Befestigungsteil 3 abgewandten Seite. Eine im Grundkörper 50 bewegbar gehaltene Klaue 51 bildet ein Gegenstück zur festen Klaue 500. Die bewegbare Klaue 51 ist mittels eines federbelasteten Druckknopfes 52, welcher aus einer Oberseite des Grundkörpers 50 herausragt, betätigbar. Mittels dieser zweiten Befestigungseinheit, nämlich den zwei Klauen 500, 51, lässt sich der zweite Befestigungsteil 5 und somit die restliche Aufhängevorrichtung wie auch der Drainagebehälter 1 an der Schiene S befestigen.

Zur Messung der Füllmenge im Beutel 12, insbesondere zur Bestimmung des Gewichts, weist die Aufhängevorrichtung zwischen dem ersten und dem zweiten Befestigungsteil 3, 5 ein Wägeteil 4 auf. Eine Anzeige 9 zeigt die gemessene Füllmenge, hier in Milliliter (ml). Andere Arten von Angaben, wie z.B. Gewichtsangaben oder graphische Anzeigen eines Füllstandes oder eines Füllverlaufes, sind auch möglich.

Im Folgenden sind anhand der Figuren 4 und 5 die einzelnen Teile der erfindungsgemässen Aufhängevorrichtung detaillierter beschrieben.

Das erste, behälterseitige Befestigungsteil 3 weist einen im Wesentlichen quaderförmigen Grundkörper 30 auf. Seine dem Behälter 1 zugewandte Seite ist mit der bereits erwähnten vertikal verlaufenden ersten Nut 31 versehen. Am Boden der ersten Nut 31 sind Durchgangslöcher 34 vorhanden, welche einen Zugang zu Befestigungsschrauben im nachfolgenden Wägeteil 4 ermöglichen. Auf der oberen Seite des Grundkörpers 30 ist mindestens ein Befestigungsloch, hier sind zwei Befestigungslöcher 33 vorhanden. Sie befinden sich in einem Randbereich der oberen Seite und dienen zur festen Verbindung mit einem Wägeelement 6, hier einer Wägezelle. Diese weist hierfür entsprechende Befestigungslöcher 60 auf. Vorzugsweise wird das behälterseitige Befestigungsteil 3 mit der Wägezelle 6 verschraubt. Andere Befestigungsarten sind jedoch auch möglich.

Auf der dem Behälter 1 abgewandten Seite des Grundkörpers 30 weist das behälterseitige Befestigungsteil 3 in seinem unteren Bereich einen ersten Vorsprung 32 auf, welcher dem zweiten, schienenseitigen Befestigungsteil 5 zugewandt ist. Die genannte abwandte Seite des Grundkörpers 30 ist ansonsten vorzugsweise plan und in Gebrauchslage in vertikaler Richtung verlaufend ausgebildet.

Das Wägeteil 4 weist ebenfalls einen im Wesentlichen quaderförmigen Grundkörper 40 mit einer zweiten Nut 42 auf, welche in horizontaler Richtung verläuft. In dieser zweiten Nut 42 ist die Wägezelle 6 gehalten, wobei die Wägezelle 6 der zweiten Nut 42 mindestens an einem Ende vorsteht und an dieser Seite mit dem behälterseitigen Befestigungsteil 3 verbunden ist. An ihrem anderen Ende ist die Wägezelle 6 mit dem Grundkörper 40 des Wägeteils 4 fest verbunden. Der Grundkörper 40 weist hierfür auf seiner Oberseite, vorzugsweise in einem Randbereich, Befestigungslöcher 43 auf. Auch hier erfolgt die feste Verbindung zwischen Grundkörper 40 und Wägezelle 6 vorzugsweise durch Verschrauben, wobei auch hier andere Arten von Befestigungen möglich sind.

Als Wägezelle 6 lassen sich bekannte Mittel einsetzen. Es handelt sich hierbei üblicherweise um Messmittel mit Dehnungsmessstreifen. Die Bezugsziffer 61 bezeichnet die Kabel, welche von der Wägezelle 6 in eine nicht dargestellte Elektronik und gegebenenfalls zu einer elektrischen Energiequelle führen. Es ist auch möglich, die gesamte oder einen Teil der Elektronik im Wägeteil 4 anzuordnen. In bevorzugten Ausführungsformen erfolgt die Datenübermittlung nicht nur an einen Display, sondern auch an externe Empfänger. In anderen Ausführungsformen fehlt ein Display. Des Weiteren ist es möglich, das Wägeteil mit einem akustischen Signalgeber auszustatten, welcher nach Massgabe des gemessenen Füllwertes aktivierbar ist. Anstelle der Wägezelle 6 kann auch ein Federelement mit Wegmessung, ein Piezosensor oder ein Drucksensor mit Linearführung eingesetzt werden.

An der der Nut 42 abgewandten Seite des Grundkörpers 40 ist ein in vertikaler Richtung verlaufender Nutstein 41 vorhanden. Vorzugsweise ist er einstückig am Grundkörper 40 angeformt. Dieser Nutstein 41 lässt sich in Eingriff mit einer dritten Nut 53 bringen, welche im Grundkörper 50 des zweiten, schienenseitigen Befestigungsteils 5 angeformt ist. Diese dritte Nut 53 endet an ihrer unteren Seite in einer doppelten Stufe, wobei dem oberen Stufenabsatz ein federbelasteter Bolzen 58 vorsteht, welcher in vertikaler Richtung entgegen der Federkraft verschiebbar ist. Auf diesem Bolzen 58 liegt das Wägeteil 4 auf. Die Federkraft muss dabei so gross gewählt sein, dass der Bolzen 58 und somit die Auflagefläche des Wägeteils 4 bei einer normalen Belastung, d.h. bei normaler Befüllung des Drainagebehälters, nicht nach unten gedrückt wird.

Am seitlichen Ende dieses oberen Stufenabsatzes, welches dem Bolzen 58 gegenüberliegt, ist eine seitliche Wand 56 hochgezogen. Vorzugsweise ist sie zusammen mit den Stufen einstückig ausgebildet. Sie weist ein durchgehendes Gewindeloch 57 auf. Eine durch dieses Gewindeloch eingedrehte Schraube, welche hier nicht dargestellt ist, greift in eine seitliche, vertikale Nut des Wägeteils 4 ein und fixiert so das Wägeteil 4 am schienenseitigen Befestigungsteil 5. Dabei ist das Wägeteil 4 in vertikaler Richtung entlang der Nut/Nutsteinverbindung nach wie vor verschiebbar. Ist keine Schraube eingeführt, so ist das Wägeteil 4 auch im Gebrauch von Hand ohne Werkzeuge vom schienenseitigen Befestigungsteil 5 lösbar.

Ist die doppelte Stufe ein separates Bauteil, so lässt sie sich beispielsweise mittels einer Schraubverbindung mit dem Grundkörper 50 des schienenseitigen Befestigungsteils 5 verbinden. Das entsprechende Gewindeloch 59 ist in Figur 4 gut erkennbar. Diese Variante hat den Vorteil, dass als Grundkörper 50 des schienenseitigen Befestigungsteils 5 die bekannten Aufhängevorrichtungen verwendet werden können. Es ist jedoch auch möglich, den Grundkörper 50 zusammen mit der doppelten Stufe und der Seitenwand 56 einstückig auszubilden.

Der untere Stufenabsatz des zweiten, schienenseitigen Befestigungsteils 5 bildet einen zweiten Vorsprung 55, welcher sich im zusammen gefügten Zustand der einzelnen Teile bis zum ersten, behälterseitigen Befestigungsteils 3 erstreckt und beabstandet unterhalb des ersten Vorsprungs 32 des behälterseitigen Befestigungsteils 3 zu liegen kommt. Der Abstand wird durch den Bolzen 58 bestimmt.

Die zwei Befestigungsteile 3, 5 und das Wägeteil 4 sind vorzugsweise im Wesentlichen aus Kunststoff gefertigt, vorzugsweise im Spritzgussverfahren.

Im bestimmungsgemässen Gebrauch ist die Aufhängevorrichtung zusammen gefügt. Das schienenseitige Befestigungsteil 5 wird an die Schiene S geklemmt und der Behälter 1 wird an das behälterseitige Befestigungsteil 3 gehängt. Wird der Behälter, genauer der Beutel 12, mit abgesaugter Körperflüssigkeit gefüllt, so wird auch das behälterseitige Befestigungsteil 3 nach unten gedrückt. Der schienenseitige Befestigungsteil und das Wägeteil 4 bleiben davon jedoch unbeeinflusst. Dadurch wird die Wägezelle 6 gebogen und ihre Biegung ergibt einen Messwert für den Füllstand.

Bei einer Überbelastung des ersten, behälterseitigen Befestigungsteils 3 reicht die Federkraft des Bolzens 58 nicht mehr aus, um das Wägeteil 4 in Position zu halten. Dieses wird ebenfalls nach unten gedrückt, jedoch maximal bis der Vorsprung 32 des behälterseitigen Befestigungsteils 3 auf dem Vorsprung 55 des schienenseitigen Befestigungsteils 5 aufliegt.

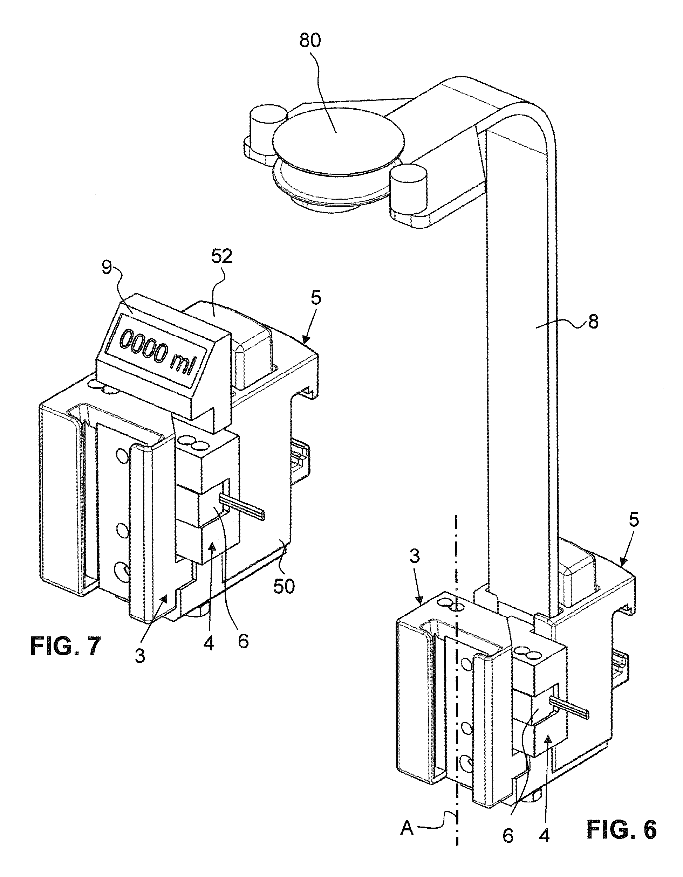

In Figur 6 ist erkennbar, wie der Bügel 8 der Zugentlastung in die Aufhängevorrichtung integriert ist. Er kann in einer einfachen Ausführungsform fest und im Gebrauch nicht lösbar mit dem Wägeteil 4 oder dem schienenseitigen Befestigungsteil 5 verbunden sein. Vorzugsweise ist jedoch hinter der dritten Nut 53 des schienenseitigen Befestigungsteils 5 eine ebenfalls vertikal verlaufende vierte Nut 54 vorhanden, in welche ein freies Ende des Bügels 8 eingeschoben und der Bügel 8 so gehalten werden kann. Eine Aufhängeachse A, welche durch die Aufhängevorrichtung definiert ist, ist ebenfalls dargestellt. Verläuft die Schiene S horizontal, so verläuft die Aufhängeachse A vertikal. Ansonsten bildet sie einen Winkel zur Vertikalen. Die oben beschriebenen Richtungen der einzelnen Teile beziehen sich auf den Fall, in welcher die Aufhängeachse A vertikal verläuft. Ansonsten sind die beschriebenen Richtungen parallel zur Aufhängeachse A zu verstehen.

In der Figur 7 ist erkennbar, dass auch der Display 9 aufgesetzt ist. Er kann an irgendeinem der beschriebenen Teile befestigt sein.

Ferner kann ein Neigesensor 7 vorhanden sein, welcher dazu dient, eine Schiefstellung des

Behälters zu detektieren. Er kann ein drohendes Umkippen des Behälters feststellen und/oder eine Schiefstellung der Schiene detektieren, damit entsprechende Korrekturen bei der Messung eingebracht werden können. Der Neigesensor ist vorzugsweise möglichst nahe am Behälter angeordnet, beispielsweise im Grundkörper 30 des behälterseitigen Befestigungsteils 3. Er kann auch in die Elektronik integriert sein. Beispielsweise ist er ein Gyrosensor.

Die erfindungsgemässe Aufhängevorrichtung ermöglicht mit einfachen Mitteln eine Bestimmung einer Füllmenge eines Drainagebehälters, insbesondere eines Drainagebehälters mit Drainagebeutel.

**BEZUGSZEICHENLISTE**

| | | | |
|---|---|---|---|
| 1 | Drainagebehälter | | |
| 10 | Aussenbehälter | 5 | schienenseitiges Befestigungsteil |
| 11 | Deckel | 50 | Grundkörper |
| 110 | Drainageanschluss | 500 | feste Klaue |
| 111 | Filterabdeckung | 51 | bewegbare Klaue |
| 112 | serieller Anschluss | 52 | Druckknopf |
| 113 | Befestigungsstutzen | 53 | dritte Nut |
| 114 | Handgriff | 54 | vierte Nut |
| 12 | Beutel | 55 | zweiter Vorsprung |
| 13 | Befestigungsvorsatz mit Nutstein | 56 | seitliche Wand |
| 14 | Federelement | 57 | Gewindeloch |
| 140 | Rückhaltenase | 58 | federbelasteter Bolzen |
| | | 59 | Durchgangsloch |
| 2 | Drainageschlauch | | |
| | | 6 | Wägeelement |
| 3 | behälterseitiges Befestigungsteil | 60 | Befestigungsloch |
| 30 | Grundkörper | 61 | Kabel |
| 31 | erste Nut | | |
| 32 | erster Vorsprung | 7 | Neigesensor |
| 33 | erstes Befestigungsloch | | |
| 34 | Durchgangslöcher | 8 | Bügel |
| | | 80 | Umlenkrolle |
| | | 81 | Führungsstift |
| 4 | Wägeteil | | |
| 40 | Grundkörper | 9 | Display |
| 41 | Nutstein | | |
| 42 | zweite Nut | A | Aufhängeachse |
| 43 | zweites Befestigungsloch | S | Schiene |
| 44 | Durchgangsloch | | |

## Patentansprüche

1. Aufhängevorrichtung eines Drainagebehälters (1), welcher zum Auffangen von abgesaugter Körperflüssigkeit dient, wobei die Aufhängevorrichtung auf einer ersten Seite eine erste Befestigungseinheit (31) zum Anhängen und Befestigen des Drainagebehälters (1) an die Aufhängevorrichtung aufweist und wobei die Aufhängevorrichtung auf einer zweiten Seite eine zweite Befestigungseinheit (500, 51) zum Anhängen und Befestigen der Aufhängevorrichtung an eine Schiene aufweist, **dadurch gekennzeichnet, dass** die erste Befestigungseinheit (31) in einem ersten Befestigungsteil (3) und die zweite Befestigungseinheit (500, 51) in einem zweiten Befestigungsteil (5) angeordnet ist und dass die Aufhängevorrichtung ein Wägeteil (4) mit einem Wägeelement (6) umfasst, wobei das Wägeteil (4) mit dem ersten Befestigungsteil (3) und dem zweiten Befestigungsteil (5) verbunden ist oder verbindbar ist, um mit dem Wägeelement (6) eine relative Verschiebung zwischen dem ersten und dem zweiten Befestigungsteil (3, 5) zu messen, so dass eine Füllmenge der abgesaugten Körperflüssigkeit im Drainagebehälter (1) bestimmbar ist.

2. Aufhängevorrichtung nach Anspruch 1, wobei die erste Befestigungseinheit (31) zur Erstellung einer bei bestimmungsgemässem Gebrauch der Aufhängevorrichtung lösbaren Verbindung mit dem Drainagebehälter (1) ausgebildet ist und/oder wobei die zweite Befestigungseinheit (500, 51) zur Erstellung einer bei bestimmungsgemässem Gebrauch der Aufhängevorrichtung lösbaren Verbindung mit der Schiene ausgebildet ist.

3. Aufhängevorrichtung nach einem der Ansprüche 1 bis 2, wobei das erste Befestigungsteil (3) und das Wägeteil (4) eine fest miteinander verbundene und bei bestimmungsgemässem Gebrauch der Aufhängevorrichtung nicht zur Trennung voneinander vorgesehene Einheit bilden.

4. Aufhängevorrichtung nach Anspruch 3, wobei das zweite Befestigungsteil (5) eine bei bestimmungsgemässem Gebrauch der Aufhängevorrichtung lösbare Verbindung mit dem Wägeteil (4) bildet.

5. Aufhängevorrichtung nach Anspruch 3, wobei das zweite Befestigungsteil (5) und das Wägeteil (4) eine fest miteinander verbundene und bei Gebrauch nicht zur Trennung voneinander vorgesehene Einheit bilden, wodurch das erste Befestigungsteil (3), das Wägeteil (4) und das zweite Befestigungsteil (5) eine bei Gebrauch nicht zur Trennung voneinander vorgesehene Einheit bilden.

6. Aufhängevorrichtung nach einem der Ansprüche 1 bis 6, wobei das Wägeelement (6) einen ersten Bereich aufweist, welcher fest mit dem ersten Befestigungsteil (3) verbunden ist, und wobei das Wägeelement (6) einen zweiten Bereich aufweist, welcher fest mit dem Wägeteil (4) verbunden ist.

7. Aufhängevorrichtung nach einem der Ansprüche 1 bis 6, wobei das zweite Befestigungsteil (5) einen Vorsprung (55) aufweist, welcher sich bis zum ersten Befestigungsteil (3) erstreckt und welcher bei einer Gewichtsüberlast zur Auflage des ersten Befestigungsteils (3) dient.

8. Aufhängevorrichtung nach einem der Ansprüche 1 bis 7, wobei sie ferner eine Zugentlastung (8) für einen mit dem Drainagebehälter (1) verbundenen Drainageschlauch (2) zum Absaugen der Körperflüssigkeit aufweist.

9. Aufhängevorrichtung nach Anspruch 8, wobei die Zugentlastung einen Bügel (8) aufweist, welcher vom Wägeteil (4) und/oder vom zweiten Befestigungsteil (5) getragen ist.

10. Aufhängevorrichtung nach einem der Ansprüche 1 bis 9, wobei sie eine Aufhängeachse (A) zur Aufhängung des Drainagebehälters (1) definiert und wobei sie einen Neigungssensor aufweist zur Feststellung eines Winkels zwischen der Aufhängeachse (A) und einer Vertikalen im Raum, in welchem sich die Aufhängevorrichtung befindet.

11. Aufhängevorrichtung nach einem der Ansprüche 1 bis 10, wobei sie ferner einen Display (9) zur Anzeige der bestimmten Füllmenge aufweist.

12. Aufhängevorrichtung nach einem der Ansprüche 1 bis 11, wobei sie ferner eine Elektronikeinheit zur Übertragung der bestimmten Füllmenge an einen externen Empfänger aufweist.

13. Befestigungseinrichtung einer Aufhängevorrichtung gemäss einem der Ansprüche 1 bis 12, wobei die Befestigungseinrichtung ein erstes Befestigungsteil (3) mit einer ersten Befestigungseinheit (31) zum Anhängen und Befestigen des Drainagebehälters (1) an die Aufhängevorrichtung aufweist, **dadurch gekennzeichnet, dass** die Befestigungseinrichtung ein Wägeteil (4) mit einem Wägeelement (6) aufweist, dass das Wägeteil (4) mit dem ersten Befestigungsteil (3) verbunden ist, und dass das Wägeteil (4) mit einem zweiten Befestigungsteil (5) zwecks Verbindung mit einer Schiene verbindbar ist, um mit dem Wägeelement (6) eine relative Verschiebung zwischen dem ersten und zweiten Befestigungsteil (43, 5) zu messen, so dass eine Füllmenge der abgesaugten Körperflüssigkeit im Drainagebehälter (1) bestimmbar ist.

14. Zweites Befestigungsteil einer Aufhängevorrichtung gemäss einem der Ansprüche 1 bis 12, wobei das zweite Befestigungsteil (5) eine zweite Befestigungseinheit (500, 51) aufweist zur Verbindung der Aufhängevorrichtung an einer Schiene, **dadurch gekennzeichnet, dass** das zweite Befestigungsteil (5) einen Gewichtsüberlastschutz aufweist, welcher durch einen verschiebbaren federbelasteten Bolzen (58) zum Tragen des Wägeteils (4) gebildet ist.

15. Drainagesystem mit einem Drainagebehälter und mit einer Aufhängevorrichtung nach einem der Ansprüche 1 bis 12, wobei der Drainagebehälter (1) einen Aussenbehälter (10), einen Beutel (12) zur Aufnahme im Aussenbehälter (10) und einen den Aussenbehälter (10) dicht verschliessenden Deckel (11) aufweist, wobei der Beutel (12) dicht mit dem Deckel (11) verbunden ist und gemeinsam mit dem Deckel (11) vom Aussenbehälter (10) entfernbar ist und wobei der Aussenbehälter (10) ferner eine Befestigungseinheit (13) zur Verbindung mit der ersten Befestigungseinheit (31) des ersten Befestigungsteils (3) aufweist.
